Europäisches Patentamt

European Patent Office

Office européen des brevets

(19)

(11) Publication number: **0 153 405**

**B1**

(12) **EUROPEAN PATENT SPECIFICATION**

(45) Date of publication of patent specification: **18.05.88**

(51) Int. Cl.⁴: **C 12 M 1/12,** C 12 M 3/00, C 12 M 1/02, C 12 M 1/04

(21) Application number: **84903320.4**

(22) Date of filing: **22.08.84**

(86) International application number: **PCT/US84/01350**

(87) International publication number: **WO 85/01062 14.03.85 Gazette 85/07**

(54) CELL CULTURE SYSTEM.

(30) Priority: **31.08.83 US 527997**

(43) Date of publication of application: **04.09.85 Bulletin 85/36**

(45) Publication of the grant of the patent: **18.05.88 Bulletin 88/20**

(84) Designated Contracting States: **AT BE CH DE FR GB LI LU NL SE**

(56) References cited:
EP-A-0 120 285
GB-A-1 128 181
US-A-4 416 993

(73) Proprietor: **CELL ENVIRONMENTAL SYSTEMS, LIMITED**
**32 Valleyview 1 Drive**
**Merrimack, NH 03054 (US)**

(72) Inventor: **BERRY, Eric, Scott**
**32 Valleyview Drive 1**
**Merrimack, NH 03054 (US)**
Inventor: **DANTI, Bernard, Richard**
**73 Bloomfield Street**
**Lexington, MA 02173 (US)**

(74) Representative: **Wiegerink, Franciscus Johannes et al**
**Nederlandsch Octrooibureau Johan de Wittlaan 15 P.O. Box 29720**
**NL-2502 LS Den Haag (NL)**

Courier Press, Leamington Spa, England.

EP 0 153 405 B1

## Description

This invention relates to a method and apparatus for the culture of cells. The cells to be cultured in this invention are prokaryotic and eukaryotic cells such as bacteria, yeast, plant, animal and human cells. These cells may be derived in any manner, that is, isolated from nature, mutated, in the naturally-occurring form, genetically engineered or modified, transformed or non-transformed, hybrids formed by fusion between portions of cells or whole cells of the same or different species. These cells may be attached to the substrate, grown in suspension, or in suspension attached to another substrate, such as microcarrier bead. The cultures may consist of a single cell line or a plurality of cell lines of the same or different species.

Conventionally, cells have been attached to and grown on the interior surface of glass or plastic roller bottles or tubes or on culture plates. This approach does not permit high-density growth of cells and requires large amounts of nutrient medium, in most cases. Use of these systems is also labor intensive.

U.S. Patents 4,189,534 and 4,266,032 describe the growth of cells on tiny beads referred to in the art as microcarrier. Microcarrier systems have several disadvantages, such as cell damage from collision of beads, mingling of the desired cell product with the nutrient medium, cell fragments and other contaminants, and the difficulty of achieving continuous production of the desired product.

It is also known that cells may be grown on or near hollow or solid fibers. See U.S. Patents 3,883,393; 3,997,396; and 4,087,327; 4,220,725; 4,391,912 also J. Feder and W. R. Tolbert, "The Large-Scale Cultivation of Mammalian Cells", Scientific American, Volume 248, Number 1 and Amicon Technical Data Publication Number 442 C, "Vitafiber (TM) Artificial Capillary Systems for Tissue Culture". Typically the nutrient medium is passed through the hollow fibers and diffuses through the lumen thereof into the cell growth space and extracapillary space bounded by an envelope. These capaillaries are susceptible to mechanical vibration and shock. The location of the cells is not closely constrained and the cells most distal from the fibers may not be well nourished. Large volumes of nutrient are typically utilized.

U.S. Patent 4,225,671 describes a cell culture apparatus in which a stack of parallel flat membranes defines separate, typically alternating, cell culture medium, and cell growth spaces. These membranes are spaced widely apart (2 mm), so excessive quantities of nutrient must be provided to ensure the nourishment of the cells most distal from the membrane. Since the membranes are supported only at their edges, the maximum surface area of each membrane is limited by the tensile strength of the membrane. Thus production capacity is limited. In addition the membrane is not designed to restrict the flow of the cell product into the nutrient medium and the product is collected in that medium. Because the cell product is mingled with the culture medium, product, recovery and purification is complex and results in a low yield of product.

U.S. Patent 3,948,732 shows a spacer structure which is convoluted to offer internal support to the substrate surfaces. It does not, however, recognize the importance of a fluid path separate from that of the nutrient medium.

One object of the invention is to provide a closed sterile system for cell culture in order to prevent both exposure of personnel to the cells and the contamination or infection of the cell culture by wild strains entering from the outside environment.

Another object of the invention is to provide a system for cell culture which more closely approximates a continuous system than those known previously, thus increasing the average or steady-state output of product and minimizing opportunities for contamination.

Another object of the invention is to simulate the supply of nutrient to and exchange of gases by cells in vivo, wherein cells are typically less than 200 μm from the capillaries, the sources of nutrients and respiratory gases.

Another object of the invention is to provide a cell culture assembly in which the maximum distance between a cell and the nearest source of nutrients and dissolved gases approximates that typical of the cells in vivo.

Another object of the invention is to provide a cell culture assembly which can be economically manufactured.

Another object of the invention is to provide a cell culture assembly and cell culture system which lends itself to the growth of cells at high densities.

Another object of the invention is to provide a cell culture assembly and cell culture system in which fluctuations in cell growth and production of the desired product are reduced.

Another object of the invention is to provide a cell culture system in which nutrient supply, heat transfer, gas transfer, cell growth and product collection functions are integrated.

Another object of the invention is to provide a cell culture system in which those functions are performed by separate modules of similar construction, and thus more economical to fabricate.

Another object of the invention is to provide multi-layered modules for cell culture and related purposes which can be assembled by lamination into an essentially rigid block structure.

Another object of the invention is to provide a cell culture assembly and cell culture system in which the nutrient medium circulation path is independent from the path carrying the cell product, metabolic products from the component of the culture medium and the cells themselves.

Another object of the invention is to provide a cell culture system in which the cells' environmental conditions are continuously monitored and any departures from the desired range of value in any environmental parameter are automatically indicated and corrected.

Another object of this invention is to provide a laminated structure for the fluid paths such that high strength and reliability are attained.

Another object of this invention is to provide a laminated structure that may be increased in size to accommodate large scale cell culture.

Another object of this invention is to provide a cell culture assembly which does not require large volumes of medium.

The aforesaid objects as well as other objects of the present invention will be made more apparent by the following description, claims and drawings.

Summary of invention

In accordance with these objects, the cell culture system comprises a series of connected modules which provide a favorable location for cell growth and maintain the environment of the cells so as to promote continued production of the desired product. Typically, the modules will be heat transfer, gas transfer, cell culture and cell-product separation modules. Multiple separate and independent circulation loops exist within the cell culture system. In a given module only two of these circulation loops are operational. Means are provided for continuous circulation of nutrient culture medium through all of these modules (except for the cell-product separation module). In the second circulation path separate and independent means (secondary or auxiliary loops) exist for (a) the passage of gases through the gas transfer module, (b) the passage of heat through the heat transfer module, (c) the introduction of cells into the cell culture module, and (d) the withdrawal of product from the cell growth module into the cell-product separation module. In addition, a separate and independent means exists for the transfer of the separator cell product(s) into a collection vessel to be held for further processing.

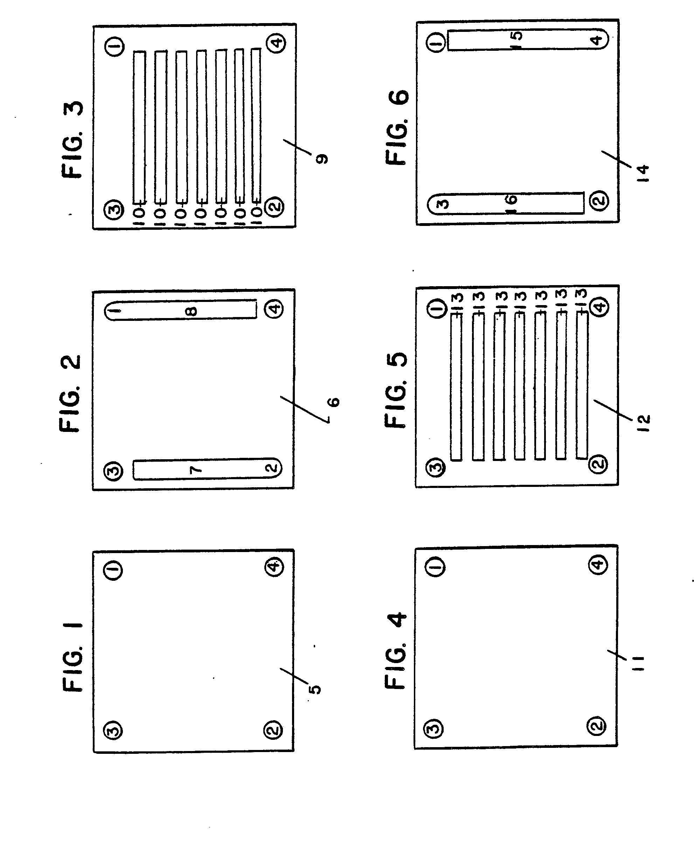

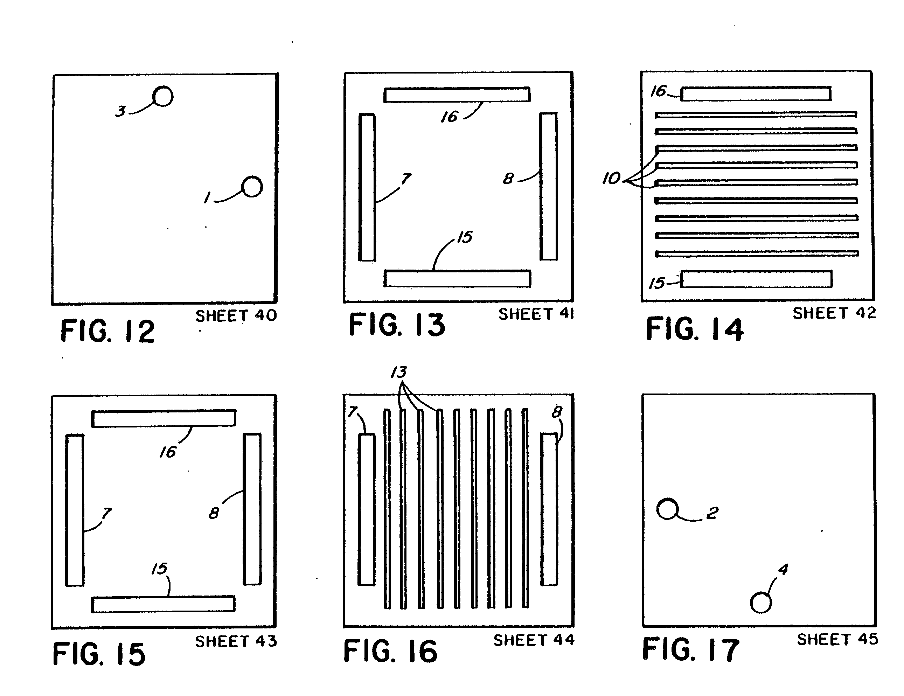

The modules are preferably fabricated in a similar manner to achieve similar structures. The common structure of the preferred modules is described below as the basic module. The various modules differ from the basic module in the nature of the separator sheets 11 of Figure 4 or sheets 43 of Figure 15, and in the nature of the fluids circulated in the auxiliary circulation system. All of the modules except the cell-product separation module are connected and serviced by the nutrient medium circulatory system.

The modules of the present invention provide a large surface area, relative to the volume occupied by the modules, for cell growth and or attachment. The modules of the present invention are sterile, disposable, and replaceable. In accordance with one of the features of the system there is provided a sterile, disposable temperature control module. This feature allows a constant temperature fluid to circulate on one side of the non-porous membrane to control the temperature of the growth medium on the opposite side of the membrane without contact or contamination. In accordance with another feature of this invention, there is provided another sterile, disposable module for the exchange of gases with the growth medium by diffusion of the gases across either a hydrophobic or hydrophilic membrane. In accordance with another feature of this system, there is provided another sterile, disposable module to provide an environment and source of suitable growth medium. The cells are then cultivated on the opposite side of the membrane free from many of the contaminates in the growth medium. In accordance with another feature of this invention, there is provided another sterile, disposable module that incorporates a microporous filter to retain any free cells in the case of substrate attached cells or in the case of suspension cells to retain the free mobile cell culture and provide a closed loop thus allowing circulation back through the growth module. The soluble products are then eluted off the opposite side. In accordance with another feature of the system the available contact surface area of the modules may be varied from 200 sq cm to 80,000 sq cm. In accordance with another object of this system there is provided a means for growing cells in a totally enclosed environment to decrease the probability of contamination of the external environment by cells or cell products, and to decrease the probability of contamination of the cellular environment by foreign particulate matter or biological organisms. This system which is comprised of individual disposable modules also provides a means for disposing of individual contaminated units in an efficient manner which minimizes the threat to the environment. Further, since the system allows the culture of high densities of cells in a small volume the necessity for handling large numbers of culture vessels and the medium required to feed them is reduced resulting in a lowered risk of compromising the laboratory and surrounding environment. In accordance with another object of this invention there is provided a selective semi-permeable membrane to separate the cell nutrient flow path from the cell growth and cellular production path and thus permits the collection of cell product in a more highly concentrated form free of certain nutrient medium components which would dilute the cell product were the two pathways common. In accordance with another object of this invention there is provided a means for the collection of the concentrated cell product that permits less complicated and less costly methods to be used in the recovery and purification of the product because smaller volumes must be processed. In addition the use of a highly concentrated starting material for recovery and purification generally promotes more efficient recovery of final product in terms of purity and recovery.

In accordance with another object of this invention there is provided a means for periodic reversal of the nutrient medium flow and cell production collection flow to provide more efficient exchange of nutrient medium and cell waste products and thereby permit growth of greater cell numbers and thus increase the amount of product produced per unit of cell growth area.

In accordance with another object of this invention there are provided assemblies wherein the boundaries of the channels constrain the cells and fluids in such a manner that the cells are never more than 200 μm from the nearest source of nutrients.

Other features and benefits of the invention will be seen as the description of each module progresses, in conjunction with the following drawings.

Fig 1—Cover plates top view
Fig 2—Auxiliary primary sheet top view
Fig 3—Auxiliary secondary sheet top view
Fig 4—Separator sheet top view
Fig 5—media secondary sheet top view
Fig 6—Media primary sheet top view
Fig 7—Pictorial view basic module construction
Fig 8—Schematic view of total system
Fig 9—Cross-section part of Fig 7
Fig 10—Schematic of microprocessor
Fig 11—Pictorial view basic module with perpendicular circulation
Fig 12—Top cover plate top view
Fig 13—Media and Auxiliary spacer sheet
Fig 14—Auxiliary capillary sheet
Fig 15—Separator sheet
Fig 16—Media capillary sheet
Fig 17—Bottom cover sheet top view
Fig 18—Exploded view basic module with perpendicular circulation

Detailed description of invention
Basic module

The basic module preferably has a large number of narrow parallel channels which are preferably formed by the superposition of the various sheets (Figures 1—6) of the assembly, as more fully set forth below.

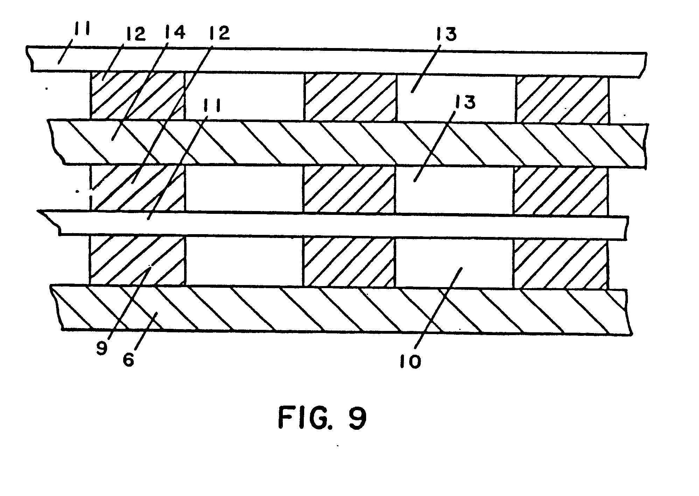

In one embodiment of the invention, a secondary sheet 9, as shown in Figure 3, has a plurality of narrow, parallel slots 10. This sheet is disposed between a separator sheet 11 (Figure 4) and a primary sheet 6 (Figure 2). As can be seen in Figure 9, these three sheets cooperate to bound a plurality of channels 10 in sheet 9 whose depth is that of sheet 9. These channels 10 will be referred to by reason of their function, as auxiliary capillaries. The number, spacing, width and depth of these auxiliary capillaries is preferably chosen in view of the function of the module in which they appear. The result is that numerous parallel channels are formed on each side of the separator sheets. The flow through the capillaries is sealed from and independent from the flow on opposite side of each separator sheet 11; although, the nature of the separator sheet 11 may allow exchange of appropriate and desired substances between the chambers. Similarly, a secondary sheet 12 (Figure 5) is disposed between a separator sheet 11 and a primary sheet 14 (Figure 6) to create channels 13, the media capillaries. As can be seen in Figure 9 these three sheets cooperate to a bound a plurality of channels 13 in sheet 12, whose depth is that of sheet 12; and that said channels are separate and independent and communicate across said membrane.

The disposition of the auxiliary primary sheet 6 (Figure 2) between the secondary sheet 9 and either a cover plate 5 or another secondary sheet 11 creates two larger channels 7 and 8, referred to as the auxiliary vein 7 and the auxiliary artery 8.

Similarly, the disposition of the media primary sheet 14 between the secondary sheet 12 and either a cover plate or another secondary sheet 11 creates a media vein 15 and a media artery 16.

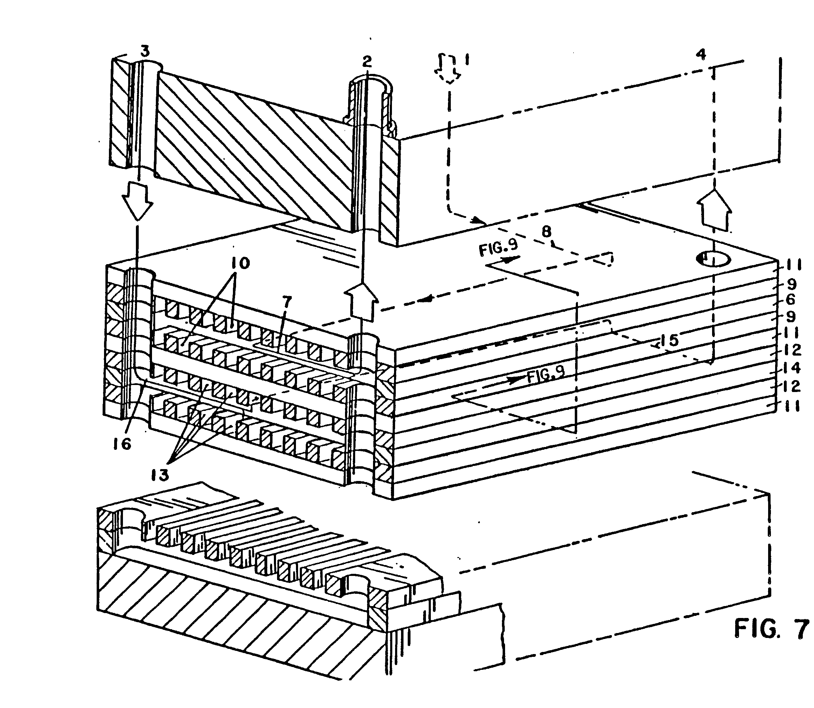

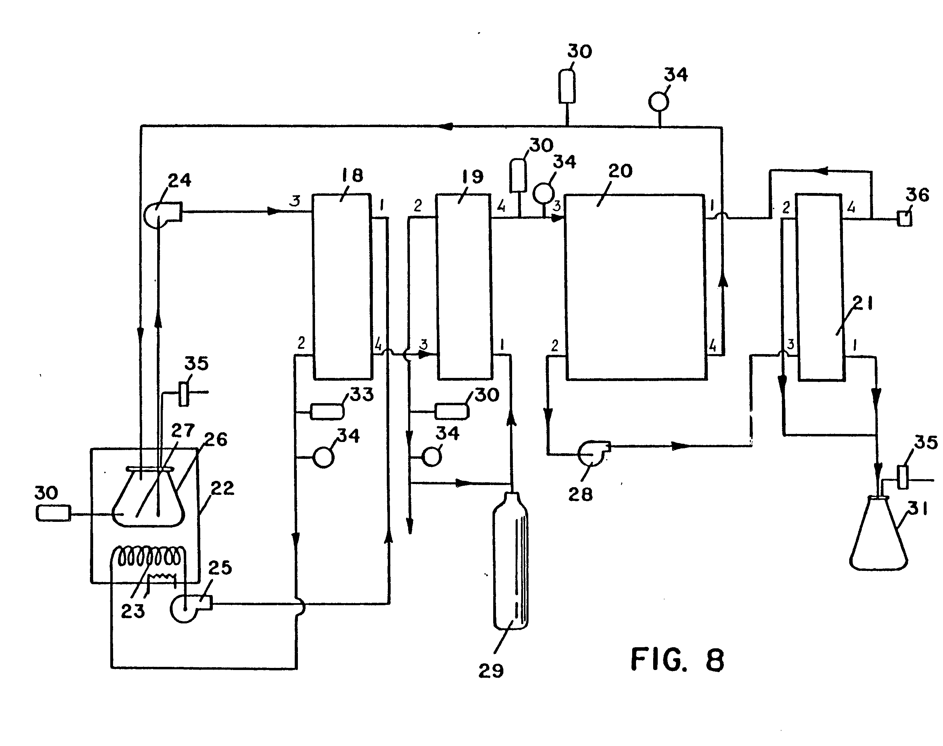

Cover plate 5, as shown in Figures 1 and 7, has as auxiliary inlet 1, an auxiliary outlet 2, a medium inlet 3, and medium outlet 4. In Figure 7, only the ports on the top cover plate are in use, while the ports on the bottom cover plate are capped. It is possible to use some top plate ports and some bottom plate ports as shown in Figure 8, moreover, the precise location of these outlets and inlets may be varied as deemed appropriate. Note that the terms "top" and "bottom" are for convenience and do not refer to the orientation of the module. The modules are preferably constructed by lamination of the sheets. It is not necessary that all of the sheets comprising the module be made from the same material.

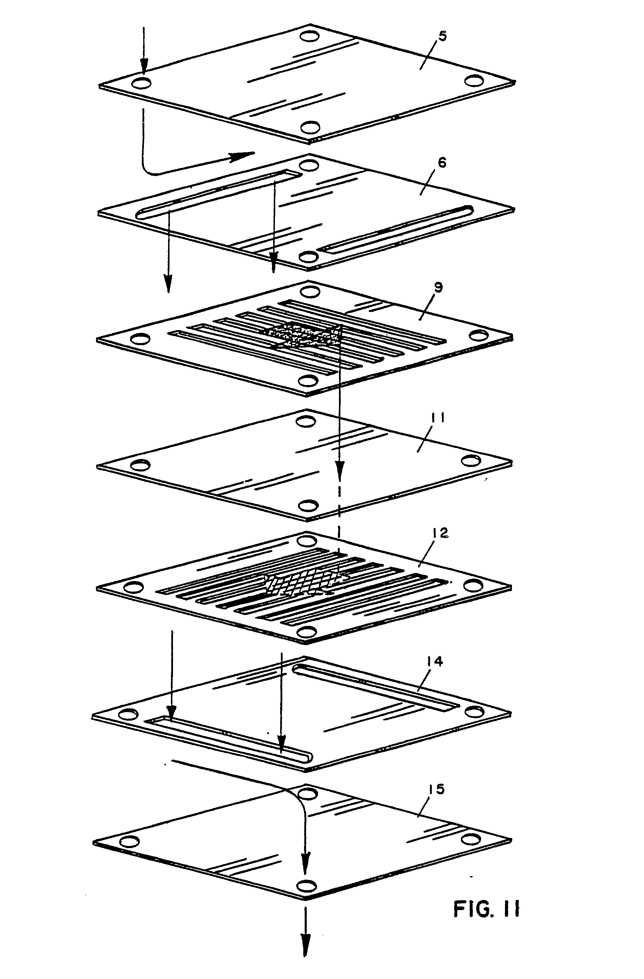

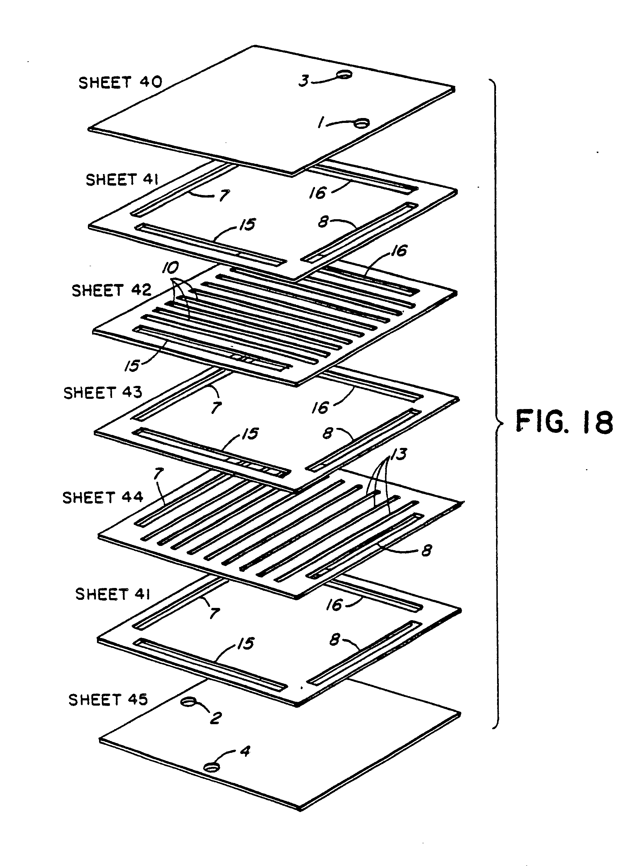

Although in the configuration shown in Figures 7, 9 and 11 the media and auxiliary capillaries are parallel to each other, it can be seen in Figure 18 that in a second embodiment these channels may run perpendicularly. In the second embodiment only one separator sheet 41 (Figure 13) may be required due to the perpendicular construction. It can be seen in Figure 13 that spacer sheet 41 contains the arteries 16, 8 and veins 15, 7 requiring two sheets, sheets 6, 14 (Figures 2, 6) in the first embodiment.

It is preferable, but not necessary, that the media and auxiliary capillary channels have the same dimensions. The channels in the appropriate sheets, may be aligned with each other, or they may be offset in the direction perpendicular to their longest dimension. This offset may increase mechanical strength. The perpendicular construction has a similar advantage.

Although in the configuration shown in Figure 18 there is only one set of media and auxiliary capillaries; it can be seen in Figure 18 that a large number of narrow parallel channels are formed by the superposition of various sheets 41—44 (Figures 13—16) as set forth below and previously described in the basic module. The superposition of an additional separator sheet 43 and media capilliary sheet 44 between the spacer sheet 41 (Figure 18) and the auxiliary capillary sheet 42 (Figure 18) provides additional media capillaries and a complete set of veins and arteries.

Similarly the superposition of an additional separator sheet 43 and auxiliary capillary sheet 42 between the media capillary sheet 44 and the spacer sheet 41 (Figure 18) provides additional

auxiliary capillaries and another complete set of veins and arteries.

As in the basic module description the flow through the capillaries is independent from and sealed from the flow on the opposite side of separator sheet 43; although, the nature of separator sheet 43 may allow the exchange of appropriate and desired substances between the chambers as shown in (Figure 8).

In the media circulatory system, media enters fluid inlet 3 sheet 40 (Figure 12) and flows into the media artery 16 of (Figures 13, 14, 15). It then is distributed through media capillaries 13 sheet 44 (Figure 16). If the separator sheet 43 (Figure 15) is a permeable membrane, then certain substances will permeate separator sheet 43 which separates media capillaries 13 (Figure 16) and the auxiliary capillaries 10 sheet 42 (Figure 14). Media leaves the media capillaries 13 through media vein 15 (Figures 13, 14, 15) and then leaves through media outlet 4 sheet 45 (Figure 17). Fluids permeating separator sheet 43 (Figure 15) are collected first in the auxiliary capillaries 10 (Figure 14) and are thence extracted from auxiliary inlet and auxiliary outlet 1, 2 (Figures 12, 17) through the arteries and veins 8, 7 (Figures 13, 15, 16).

Suitable culture media for the cultivation of various types of cells are known and may be used in the method and apparatus of this invention. Culture media typically are composed of assimilable sources of elements such as carbon, nitrogen, and phosphorous and may include amino acids and blood serum, by way of example. The composition of the culture medium may be varied from time to time to control cell metabolism and reproduction. In addition, the pH of the medium may be controlled or monitored.

The function of the auxiliary circulatory system varies from one type of module to another. Fluid enters at auxiliary inlet 1 and flows into the auxiliary arteries 8 of (Figure 2). It is then distributed by auxiliary capillaries 10 (Figure 3). The fluid is collected by the auxiliary veins 7 and withdrawn through auxiliary outlet 2.

The number of veins, arteries and capillaries in the module is proportional to the number of separator sheets 11 (Figure 4) or sheets 43 (Figure 15) in the module Typically, a single module will contain 2 to 400 separator sheets. Preferably each secondary sheet defines a minimum of 20 capillaries 10 or 13 (Figures 3, 14 and 5, 16 respectively), and these capillaries are, 7 thousandths of an inch (178 μm) deep and no more than 25 centimeters in length. The width and spacing are less important, but we have successfully used a width of 760 to 1650 μm, and a spacing of 1270 to 2540 μm. The width, depth and spacing may be varied, in response to the growth needs of the cells. The minimum dimensions are defined by the typical dimensions of a single cell of the type of cells to be cultivated. The maximum dimension can not be so great as to unduly limit the nourishment of cells at the distal wall of the cell growth spaces from the membrane. In addition, as the width is increased, the strength of the structure is weakened. The depth should be similar to the maximum distance separating cells from capillaries in vivo, and it is thought that 500 μm is the upper limit if the advantages of this structure are to be achieved. Peferably, it should be less than 200 μm, however the depth may be increased to 1000 microns to accomodate "microcarriers", or other large hybrid cells.

The terms "veins", "arteries" and "capillaries" are utilized hereto suggest the relative dimensions of these channels and the veins, whether they carry fluid into or out of the module, and to emphasize that the system and its component modules are meant to simulate certain of the characteristics of the supply of nutrients to and exchange of gases by cells in vivo.

The term fluid is here used to refer to both liquids and gases, whether pure or mixed, and to include liquids or gases carrying substances in solution or in suspension. The following will be a description of the heat transfer module, gas transfer module, cell growth module and the cell-product separation module. It should be assumed that all modules will have the structure of the basic module, except as set forth below.

Heat exchange module

In the heat exchange module (18) according to (Figure 8), the separator sheet 11 (Figure 4) or separator sheet (Figure 15) is nonporous, and preferably of polysulfone, or other suitable film with good heat transfer characteristics (greater than 11.7 J/sec per square centimeter). The auxiliary arteries, veins and capillaries conduct a heat transfer fluid, which mediates the temperature of the nutrient medium 27 in the nutrient medium capillaries 13 of module 18 as a result of heat transfer across separator sheet 11 or sheet 43.

The temperature at which the culture medium is maintained may be varied in accordance with the growth characteristics of the cells, and hence the heat transfer fluid may have either a heating effect or a cooling function. The fluid temperature is maintained in a conventional manner.

Gas exchange module

In the gas exchange module 19 according to (Figure 8) the separator sheet 11 (Figure 4) or separator sheet 43 (Figure 15), is a porous film which may be either hydrophobic or hydrophilic in nature; and which has the ability to flow at least $7*10E-6$ ml/min-sq-cm-mm Hg. The auxiliary arteries conduct life-sustaining gases into the auxiliary capillaries 10. These gases then pass across the separator sheet 11 or 43 into media capillaries 13 to be carried eventually to the cells in module 20 (Figure 8). Waste gases carried by the medium 27 from module 20 are carried eventually to module 19 where they pass across the sheet 11 or 43, into the auxillary capillaries 10 and thence are removed from the system via auxiliary veins of the gas module 19.

The gas mixture circulated by the gas transport module may be air, or an artificial mixture of various elemental and compound gases.

The term "cell culture" encompasses both conditions for cell conservation and metabolic production and conditions providing for cell growth.

## Cell growth module

In the cell culture module 20 (according to Figure 8), the separator sheet 11 Figure 4 or separator sheet 43 Figure 15 is a porous film which is hydrophilic in nature; but which has a known and selected porosity. The porosity is such to restrict the flow of substances of known molecular weights and/or charge, whose passage across the separator sheet 11 or sheet 43 from the media capillaries into the auxiliary capillaries is not desired. For example, a membrane having a 10,000 dalton nominal cutoff may be used to restrict the entrance of serum components including proteins having nominal molecular weights more than 10,000 daltons into the cell growth spaces, defined by the auxiliary capillaries. Likewise products which have a molecular weight greater than 10,000 daltons produced by the cells and secreted into the extracellular medium are restricted from entering the nutrient medium flow path. Membranes having higher or lower molecular weight cutoffs (range 1000—->1×10E7) can be used to restrict the transfer of desired molecular weight materials from adjacent chambers. Similarly different charge densities may be applied to the membranes to restrict the transfer of charged materials across adjacent membranes. Thus standard culture media containing unnecessary components may be utilized without mingling these components with the desired product.

The auxiliary artery conducts new cells suspended in extra-cellular fluid, into the auxiliary capillaries of module 20, the cell growth or culture spaces, where they may adhere to the walls of the capillaries or may remain in suspension or attached to microcarriers.

These cells are nourished by nutrients passing across sheet 11 or sheet 43 from the nutrient media capillaries. Product is removed through the auxiliary veins and does not mix with nutrient media 27. Once the cells are aseptically introduced into the cell culture assembly, other undesired cells cannot enter the cell culture spaces across the membrane from the nutrient medium spaces. Consequently, contamination of the cell culture spaces is prevented. (Of course, since the nutrient medium is continuously circulated within a closed system, it is unlikely that the medium would be contaminated in the first place.)

## Cell-product separation module

In the cell-separation module 21 according to (Figure 8) the separator sheet 11 (Figure 4) or separator sheet 43 (Figure 15) is a porous membrane which is hydrophilic in nature, and has the ability to retain particles of a known size. For example, a membrane having a pore size of 5 μm could be used to restrict cells having a mean size of greater than 5 μm to the cell growth module. This pore size would permit transfer of the cell products across the membrane into a collection means, which may be a holding vessel or additional processing apparatus. The conduit 16 of module 21 distributes fluid received from inlet 3 (sometimes referred to as media inlet 3) to the capillary channels 13 (sometimes referred to as media capillaries). The fluid 32 then moves along separator sheet 11 or 43, which retains particles greater than the rated size. Part of this fluid 32 passes through the membrane and is collected in the auxiliary channels 10 and collected in the auxiliary conduits 7 and 8 thence passed to collection means 31 from auxiliary connections 1 and 2.

## The cell culture system

Referring to Figure 8 there is shown an incubator 22 containing heat transfer fluid 23, maintained at a suitable temperature. The heat transfer fluid 23 is pumped to inlet 1 of the temperature control module 18 by pump 25 and returned to the incubator from outlet 2. Controlled gases from reservoir 29 are introduced to auxiliary inlet 1 of module 19 by pressure and exit through sensor (30, 34) from outlet 2 module 19 to reservoir 29. Located in the incubator 22 is a media reservoir 26 containing medium 27. Medium 27 is pumped using a diaphragm pump 24 to inlet 3 of the temperature control module 18 then from outlet 4 of module 18 to inlet 3 of the gas exchange module 19. Collected at outlet 4 of module 19; and fed to inlet 3 of the cell growth module 20, and finally it is collected at outlet 4 and returned to reservoir 26, through sensor block 30. The cells are introduced by opening valve 36 to inlet 1 of the cell growth module 20. The extracellular fluid which includes both product and loose cells, is pumped to inlet 3 of the cell/product separation module 21, by pump 28 from outlet 2 module 20. The loose cells and product are separated by separator 11 or sheet 43 (Figure 15) and the product is conducted to collection means 31. The remaining extracellular fluid is returned to inlet 1 module 20. Alternatively the cells themselves may be removed through a valve 36 after outlet 4 of module 21. This allows the harvest of cells to be ruptured to release product(s) not normally secreted by the cells. The product may be removed from collection means 31 through valve 35. Despite the use of the term "continuous process", it should be understood that, on occasion the culture medium and other fluids may be replaced.

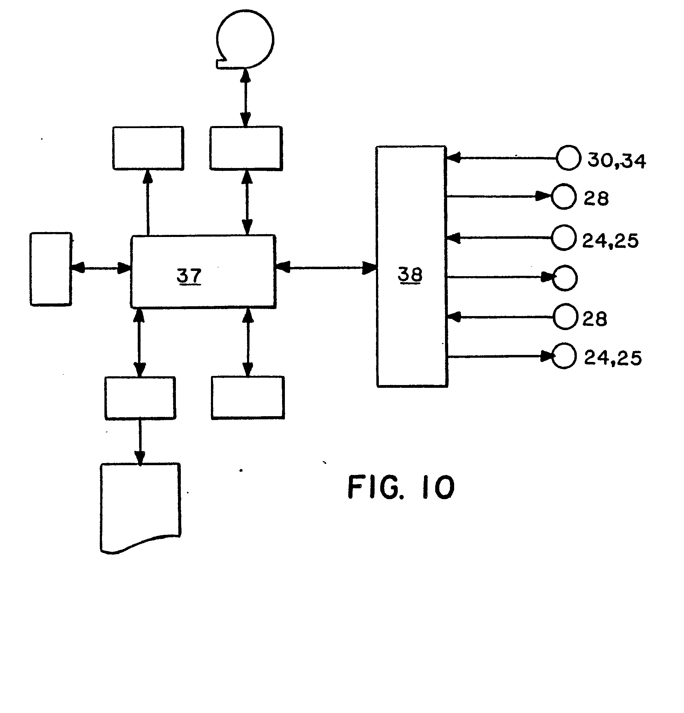

Preferably, an automatic control subsystem is provided. A microprocessor 37 of Figure 10 is connected to sensor blocks 30 and 34 via an A/D, D/A convertor 38 so as to monitor the pressures, gases, pH, temperature, and dissolved gas content and to pumps 24, 25 and 28 such that it will monitor, alarm, and react if any of the state variables enter an unacceptable range. This is especially important for preserving the safe atmosphere for the personnel using the equipment. Alarm means are connected to the microprocessor and triggered when an unacceptable state is reached. By control means connected to said pumps, it causes corrective measures to be taken. This automated system enhances the safety of the closed system by virtue of the controls, alarms and ability to shut down the system on detection of failure. This feature thus provides an additional safety factor for the personnel in the production environment.

While in Figure 7 only a single module of each type is shown in the cell culture system, it should be understood that additional modules of any type may be added to the system. In addition, it should be understood that the system may be simplified (though at a cost to its ability to function automatically for long periods of time) by omitting certain of the modules.

While a cell culture system preferably employs modules of the modular construction described herein, any of the individual modules may be used in a cell culture system which is not characterized by modularity. For example, a device having the structure of the cell growth module as described in the above may be used as a cell growth assembly in a non-modular cell culture system.

**Claims**

1. A basic module for a cell culture system of modular construction comprising

a first fluid circulation means for the circulation of culture medium through the module;

a second fluid circulation means for the circulation of another fluid; and

a separator means separating said first and second fluid circulation means

wherein each of said fluid circulation means defines a plurality of separate, narrow, closely-spaced and shallow channels into which the fluid is distributed and from which it is collected, and wherein said channels are defined by the superposition of slotted sheets and separator sheet in alternating fashion.

2. The module of claim 1 wherein said channels have a depth of less than about 1000 μm.

3. The module of claim 2 wherein said module is a cell culture module, the channels of said second fluid circulation means are cell culture spaces, into which cells are introduced and from which the metabolic products of said cells are withdrawn and wherein said separator means is a selectively permeable membrane.

4. The module of claim 2 wherein said module is a heat exchange module, said second fluid circulation means operates to circulate a heat transfer fluid through said module; and said separator means is made of a nonporous material having good heat transfer characteristics.

5. The module of claim 2 wherein said module is a gas exchange module, said separation means is made of a porous material and permits a gaseous flow, at least 7×10E-6 ml/min-sq-cm-mmHg through the pores thereof said second fluid circulation means introduces life-sustaining gases into the module and withdraws waste gases therefrom, and gases are exchanged between said first and second fluid circulation means through said membrane.

6. The module of claim 1 wherein said module is a cell and product separation unit, wherein the first fluid circulation means conveys a fluid containing product and possibly containing loose cells into the unit, the second fluid circulation means withdraws product from the unit, and the separator means is a porous, hydrophilic selectively permeable membrane capable of retaining cells and passing product.

7. A cell culture system comprising:

a continuous-loop culture medium circulation means circulating a culture medium

a heat exchange means whereby heat is transferred to or from said culture medium

a gas exchange means whereby gases are transferred to or from said culture medium and said gas circulation means;

a cell growth means communicating with said culture medium circulation means; and

means for introducing cells into said cell growth means;

a means for removing product and dead cells from said cell growth means;

a cell and product separation means communicating with said removal means; and

a product collection means communicating with said separation means,

wherein said system includes at least one module according to claim 1.

8. The cell culture system of claim 7, further comprising

sensor means for monitoring the physical and chemical state of the culture medium;

processor means for determining whether said state is acceptable; and

automatic control means for altering the operation of said culture medium circulation and heat exchange means in order to drive the state of the system toward an acceptable state.

9. The cell culture system of claim 7 in which the heat exchange means is the module of claim 4, the gas exchange means is the module of claim 5, the cell and product separation means is the module of claim 6, and the cell growth means is the module of claim 3.

10. The cell culture system of claim 9, further comprising means for reversing the flow of medium in the culture medium circulation means.

11. A cell growth assembly having a nutrient medium circulation means, and extracellular fluid circulation means, and a selectively permeable membrane separating said means, wherein each of said circulation means distribute fluid into and collect fluid out of separate narrow, closely-spaced, shallow and parallel channels, wherein said membrane separates the channels of said first circulation means from the channels of said second circulation means.

12. A cell culture assembly according to claim 11, further comprising cover plates, an auxiliary primary sheet, an auxiliary secondary sheet, a selectively permeable membrane, a nutrient medium secondary sheet, a nutrient medium primary sheet, and a bottom cover plate, in a stacked relationship wherein said cover plate and said sheets have openings defining an auxiliary inlet, an auxiliary outlet, a nutrient medium inlet, and a nutrient medium outlet, the auxiliary inlet and outlet openings in the auxiliary primary sheet are slots running perpendicular to the respective inlet and outlet; the media inlet and outlet openings in the medium primary sheets are slots running perpendicular to the respective inlets and outlets; the secondary sheets have multiple narrow slots overlapping the slots in the respective adjacent primary sheets perpendicular to both the slots in the primary sheets and to the inlets and outlets; and the membrane is not slotted except for the openings defining the inlets and outlets, and said sheets are laminated together with said cover plates to form an essentially rigid structure.

13. A method for the in-vitro biosynthesis of (a) cellular product(s) in a cell culture system according to claim 7, comprising the steps of

(a) introducing cells capable of producing the desired cellular product(s) into the cell growth means, said means being a module according to claim 3;

(b) continuously circulating culture medium separately in the culture medium of a circulation means,

(c) cultivating the cell under suitable conditions, and

(d) isolating or liberating the desired cellular product(s) from said cell growth spaces or cells, respectively.

14. The method of claim 13 wherein the wall of the cell growth spaces is such that no cell in any of said spaces is further from said membrane than about 500 µm.

15. The module of claim 3 wherein the channels of the first fluid circulation means are substantially parallel to the cell growth channels of the second fluid circulation means.

16. The module of claim 3 wherein the nutrient-carrying channels of the first fluid circulation means are substantially perpendicular to the cell growth channels of the second fluid circulation means.

17. The module of claim 3 wherein the depth of a cell culture space is less than about 200 µm.

18. The module of claim 3 wherein the molecular weight cutoff for passage through the membrane is between about 1000 and $1 \times 10E7$ daltons.

19. The system of claim 7 wherein the system is enclosed and sealed.

20. A cell culture assembly comprising a rigid structure having a plurality of cell culture spaces, and a plurality of separate culture medium spaces, each cell culture space being separated from the nearest culture medium space by a selectively permeable membrane, said culture medium spaces simulating a vascular network, and said cell culture spaces being so dimensioned and oriented that the distance between a cell in a distal region of any said spaces from said vascular network is less than or substantially similar to that typical of the tissues or organs in which a cell of the type grown is found in vivo.

**Patentansprüche**

1. Basismodul für eine Zellkulturanlage von modularer Konstruktion, umfassend
ein erstes Fluidzirkulationsmittel zur Zirkulation des Kulturmediums durch den Modul,
ein zweites Fluidzirkulationsmittel zur Zirkulation eines anderen Fluids, und
ein Trennmittel, das das erste und zweite Fluidzirkulationsmittel voneinander trennt,
bei welchem jedes der Fluidzirkulationsmittel eine Mehrzahl von getrennten, engen, eng beeinander liegenden, wenig tiefen Kanälen definiert, worin das Fluid verteilt wird und woraus es gesammelt wird, und bei welchem diese Kanäle durch die abwechselnde Überlagerung von geschlitzten Folien und Trennfolien definiert sind.

2. Modul nach Anspruch 1, bei welchem die Kanäle eine Tiefe von weniger als etwa 1000 µm aufweisen.

3. Modul nach Anspruch 2, bei welchem der Modul ein Zellkulturmodul ist, die Kanäle des zweiten Fluidzirkulationsmittels Zellkulturräume sind, worin Zellen eingeführt werden und woraus die Stoffwechselprodukte dieser Zellen ausgeführt werden, und das Trennmittel eine selektiv durchlässige Membran ist.

4. Module nach Anspruch 2, bei welchem der Modul ein Wärmeaustauschmodul ist, das zweite Fluidzirkulationsmittel bewirkt, dass ein Wärmeaustauschfluid durch den Modul zirkuliert, und das Trennmittel aus nicht porösem Material von guten Wärmeübertragungseigenschaften hergestellt ist.

5. Modul nach Anspruch 2, bei welchem der Modul ein Gasaustauschmodul ist, das Trennmittel aus porösem Material hergestellt ist und durch dessen Poren einen Gasfluss von mindestens $7 \times 10^{-6}$ ml/min.cm².Torr erlaubt, das zweite Fluidzirkulationsmittel lebenserhaltende Gase in den Modul einführt und daraus Abgase ausführt, und zwischen dem ersten und dem zweiten Fluidzirkulationsmittel Gase durch die Membran hindurch ausgetauscht werden.

6. Modul nach Anspruch 1, bei welchem der Modul eine Einheit zur Trennung von Zellen und Produkt ist, das erste Fluidzirkulationsmittel ein das Produkt und möglicherweise lose Zellen enthaltendes Fluid zur Einheit führt, das zweite Fluidzirkulationsmittel das Produkt aus der Einheit ausführt, und das Trennmittel eine poröse, hydrophile, selektive durchlässige Membran ist, die fähig ist, Zellen zurückzuhalten und das Produkt durchzulassen.

7. Zellkulturanlage, umfassend
ein Mittel zur Zirkulation eines Kulturmediums in geschlossenem Kreislauf, das ein Kulturmedium zirkulieren lässt,
ein Wärmeaustauschmittel, mit welchem Wärme zu oder aus dem Kulturmedium übertragen wird,

ein Gasaustauschmittel, mit welchem Gase zur oder aus dem Kulturmedium und dem Gaszirkulationsmittel übertragen werden,

ein mit dem Mittel zur Zirkulation eines Kulturmediums kommunizierendes Zellwachstumsmittel, und

Mittel zum Einführen von Zellen in das Zellwachstumsmittel,

ein Mittel zum Ausführen von Produkt und toten Zellen aus dem Zellwachstumsmittel,

ein mit dem Mittel zum Ausführen kommunizierendes Mittel zur Trennung von Zellen und Produkt, und

ein mit dem Mittel zur Trennung kommunizierendes Mittel zum Sammeln von Produkt,

bei welcher das System mindestens ein Modul gemäss Anspruch 1, umfasst.

8. Zellkulturanlage nach Anspruch 7, ausserdem umfassend

Fühlmittel zur Überwachung des physikalischen und chemischen Zustands der Kulturmediums,

Prozessormittel zum Feststellen, ob dieser Zustand akzeptable ist, und

automatische Steuerungsmittel zum Verändern des Betriebs des Mittels zur Zirkulation eines Kulturmediums und des Wärmeaustauschmittels, um den Zustand des Systems zu einem akzeptablen Zustand zu bringen.

9. Zellkulturanlage nach Anspruch 7, bei welcher das Wärmeaustauschmittel der Modul von Anspruch 4, das Gasaustauschmittel der Modul von Anspruch 5, das Mittel zur Trennung von Zellen und Produkt der Modul von Anspruch 6, und das Zellwachstumsmittel der Modul von Anspruch 3 ist.

10. Zellkulturanlage nach Anspruch 9, ausserdem umfassend Mittel zum Umkehren des Flusses des Mediums im Mittel zur Zirkulation eines Kulturmediums.

11. Anordnung zur Zellwaschstun mit einem Mittel zur Zirkulation eines Nährmittels, und Mittel zur Zirkulation von extrazellulärem Fluid, und eine diese Mittel trennende, selektiv durchlässige Membran, bei welcher jedes der Zirkulationsmittel Fluid in getrennte, enge, eng beeinander liegende, wenig tiefe und einander parallele Kanäle verteilt und daraus sammelt, und bei welcher die Membran die Kanäle des erste Fluidzirkulationsmittels von den Kanälen des zweiten Fluidzirkulationsmittels trennt.

12. Zellkulturanordnung nach Anspruch 11, ausserdem umfassend Deckplatten, ein primäres Hilfsblatt, ein sekundäres Hilfsblatt, eine selektiv durchlässige Membran, ein sekundäres Nährmittelblatt, ein primäres Nährmittelblatt, und eine Bodendeckplatte in aufeinandergestapelter Beziehung zueinander, bei welcher die Deckplatte und die Blätter Oeffnungen aufweisen, die einen Hilfseinlass, einen Hilfsauslass, einen Nährmitteleinlass und einen Nährmittelauslass definieren, die Hilfseinlassöffnungen und die Hilfsauslassöffnungen in dem primären Hilfsblatt als rechtwinklig zum betreffenden Einlass und Auslass laufende Schlitze ausgebildet sind, die Mediumeinlassöffnungen und die Mediumauslassöffnungen in den primären Mediumblättern als rechtwinklig zu den betreffenden Einlässen und Auslässen laufende Schlitze ausgebildet sind, die sekundären Blätter eine Mehrzahl von schmalen Schlitzen aufweisen, die mit den Schlitzen in den betreffenden benachbarten primären Blättern überlappend und rechtwinklig sowohl zu den Schlitzen in den primären Blättern wie auch zu den Einlässen und Auslässen angeordnet sind, die Membran mit Ausnahme der die Einlässe und die Auslässe definierenden Oeffnungen nicht geschlitzt ist, und die Blätter zusammen mit den Deckplatten laminiert sind, um eine im wesentlichen starre Struktur zu bilden.

13. Verfahren zur Biosynthese in vitro von einem zellulären Produkt oder zellulären Produkten in einer Zellkulturanlage gemäss Anspruch 7, umfassend die Verfahrensschritte:

(a) Einführen von Zellen, die zur Produktion des (der) gewünschten Produkts (Produkte) fähig sind, in das Zellwachtumsmittel, wobei dieses ein Modul gemäss Anspruch 3 ist,

(b) kontinuierliche Zirkulation getrennter Kulturmedia im Kulturmedium eines Zirkulationsmittels,

(c) Kultivierung der Zellen unter geeigneten Bedingungen, und

(d) Isolierung oder Befreiung des (der) gewünschten Produkts (Produkte) aus den jeweiligen Zellwachstumsräumen oder Zellen.

14. Verfahren nach Anspruch 13, bei welchem die Wandung der Zellwachstumsräume derart ausgebildet ist, dass keine Zelle in irgendeinem dieser Räume von der Membran entfernter ist als etwa 500 µm.

15. Modul nach Anspruch 3, bei welchem die Kanäle des ersten Fluidzirkulationsmittels im wesentlichen parallel zu den Zellwachstumskanälen des zweiten Fluidzirkulationsmittels angeordnet sind.

16. Modul nach Anspruch 3, bei welchem die Nährmittel führenden Kanäle des ersten Fluidzirkulationsmittels im wesentlichen rechtwinklig zu den Zellwachstumskanälen des zweiten Fluidzirkulationsmittels angeordnet sind.

17. Modul nach Anspruch 3, bei welchem die Tiefe eines Zellkulturraumes weniger als etwa 200 µm beträgt.

18. Modul nach Anspruch 3, bei welchem die Schwelle des Molekulargewichts für den Durchgang durch die Membran zwischen etwa 1000 und $10^7$ Dalton liegt.

19. Anlage nach Anspruch 7, bei welcher die Anlage eingeschlossen und abgedichtet ist.

20. Zellkulturanordnung, umfassend eine starre Struktur mit einer Mehrzahl von Zellkulturräumen und einer Mehrzahl von getrennten Kulturmediumräumen, wobei jeder Zellkulturraum vom nächstliegenden Kulturmediumraum durch eine selektiv durchlässige Membran getrennt ist, die Kulturmediumräume ein vaskuläres Netzwerk simulieren und die Zellkulturräume so dimensioniert und orientiert sind, dass der Abstand zwischen einer im distalen Bereich irgendeines dieser Räume befindlichen Zelle und dem vaskulären Netzwerk kleiner ist als derjenige oder im wesentlichen gleich ist wie derjenige, der für Gewebe oder Organe, in denen eine Zelle des kultivierten Typs in vivo gefunden wird, typisch ist.

**Revendications**

1. Module de base pour un système de culture de cellules de construction modulaire, comportant

un premier moyen de circulation de fluide pour faire circuler du milieu de culture à travers le module,

un deuxième moyen de circulation de fluide pour faire circuler un autre fluide, et

un moyen séparateur qui sépare l'un de l'autre les premier et deuxième moyens de circulation de fluide,

dans lequel chacun des moyens de circulation de fluide définit une pluralité de canaux séparés, étroits, rapprochés et peu profonds dans lesquels le fluide est réparti et dont il est recueilli, et dans lequel ces canaux sont définis par la superposition alternée de feuilles munies de fentes et de feuilles séparatrices.

2. Module selon la revendication 1, dans lequel les canaux ont une profondeur de moins d'environ 1000 µm.

3. Module selon la revendication 2, dans lequel le module est un module de culture de cellules, les canaux du deuxième moyen de circulation de fluide sont des espaces de culture de cellules dans lesquels on introduit des cellules et desquels on recueille les produits du métabolisme de ces cellules, et dans lequel le moyen séparateur est une membrane à perméabilité sélective.

4. Module selon la revendication 2, dans lequel le module est un module échangeur de chaleur, le deuxième moyen de circulation de fluide fonctionne de façon à faire circuler un fluide caloporteur à travers le module, et le moyen séparateur est en matériau non poreux présentant de bonnes propriétés de transport de chaleur.

5. Module selon la revendication 2, dans lequel le module est un module échangeur de gaz, le moyen séparateur est en matériau poreux et laisse passer à travers ses pores un flux gazeux d'au moins $7 \times 10^{-6}$ ml/min.cm².mmHg, le deuxième moyen de circulation de fluide introduit des gaz vitaux dans le module et en retire les gaz de déchet, et des gaz sont échangés à travers la membrane entre les premier et deuxième moyens de circulation de fluide.

6. Module selon la revendication 1, dans lequel le module est une unité de séparation de cellules et de produit, le premier moyen de circulation de fluide transporte vers l'unité un fluide qui contient du produit et éventuellement des cellules libres, le deuxième moyen de circulation de fluide retire du produit de l'unité, et le moyen séparateur est une membrane poreuse, hydrophile, à perméabilité sélective, capable de retenir les cellules et de laisser passer le produit.

7. Système de culture de cellules comportant un moyen de circulation de milieu de culture en boucle fermée pour faire circuler du milieu de culture,

un moyen échangeur de chaleur par lequel de la chaleur est amenée ou retirée du milieu de culture,

un moyen échangeur de gaz par lequel des gaz sont amenés ou retirés du milieu de culture et du moyen de circulation de gaz,

un moyen de croissance de cellules qui communique avec le moyen de circulation de milieu de culture,

des moyen d'introduction de cellules dans le moyen de croissance de cellules,

un moyen d'extraction de produit et de cellules mortes du moyen de croissance de cellules,

un moyen séparateur de cellules et de produit qui communique avec le moyen d'extraction, et

un moyen de recueil de produit qui communique avec le moyen séparateur,

dans lequel le système comporte au moins un module selon la revendication 1.

8. Système de culture de cellules selon la revendication 7, comportant en outre

des moyens de sondage pour surveiller l'état physique et chimique de milieu de culture,

des moyens de traitement pour déterminer si cet état est acceptable, et

des moyens de commande automatique pour modifier le fonctionnement du moyen de circulation de milieu de culture et du moyen échangeur de chaleur afin d'amener l'état du système vers un état acceptable.

9. Système de culture de cellules selon la revendication 7, dans lequel le moyen échangeur de chaleur est le module selon la revendication 4, le moyen échangeur de gaz est le module selon la revendication 5, le moyen séparateur de cellules et de produit est le module selon la revendication 6 et le moyen de croissance de cellules est le module selon la revendication 3.

10. Système de culture de cellules selon la revendication 9, comportant en outre des moyens pour renverser le flux de milieu dans le moyen de circulation de milieu de culture.

11. Assemblage pour croissance de cellules, comportant un moyen de circulation de milieu nutritif, un moyen de circulation de fluide extracellulaire, et une membrane poreuse à perméabilité sélective qui sépare ces moyens, dans lequel chacun des moyens de circulation répartit et recueille du fluide de canaux séparés, étroits, rapprochés peu profonds et parallèles, et la membrane sépare les canaux du premier moyen de circulation de fluide des canaux du deuxième moyen de circulation de fluide.

12. Assemblage pour croissance de cellules selon la revendication 11, comportant en outre des plaques de couverture, une feuille auxiliaire primaire, une feuille auxiliaire secondaire, une membrane à perméabilité sélective, une feuille secondaire de milieu nitritif, une feuille primaire de milieu nutritif et une plaque de couverture de fond en relations mutuelles d'empilement, dans lequel

la plaque de couverture et les feuilles comportent des ouvertures qui définissent une entrée auxiliaire, une sortie auxiliaire, une entrée de milieu nutritif et une sortie de milieu nutritif,

les ouvertures auxiliaires d'entrée et de sortie dans la feuille auxiliaire primaire sont des fentes courant perpendiculairement à l'entrée et à la sortie respective,

les ouvertures d'entrée et de sortie de milieu dans les feuilles de milieu primaires sont des fentes courant perpendiculairement aux entrées et sorties respectives,

les feuilles secondaires comportent de multiples fentes étroites qui se superposent aux fentes des feuilles primaires adjacentes respectives et courent perpendiculairement à la fois aux fentes des feuilles primaires et aux entrées et sorties,

la membrane ne comporte pas de fentes à l'exception des ouvertures qui définissent les entrées et sorties, et

les feuilles sont laminées ensemble avec les plaques de couverture pour former une structure essentiellement rigide.

13. Procédé de biosynthèse in vitro d'un produit cellulaire ou de produits cellulaires dans un système de culture de cellules selon la revendication 7, comportant les étapes suivantes:

(a) introduction, dans le moyen de croissance de cellules, de cellules capables de produire le ou les produits cellulaires désirés, ce moyen de croissance de cellules étant le module selon la revendication 3,

(b) circulation continue séparée de milieu de culture dans le milieu de culture d'un moyen de circulation,

(c) culture des cellules dans des conditions convenables, et

(d) isolement ou libération du ou des produits cellulaires désirés respectivement des espaces de croissance de cellules ou des cellules.

14. Procédé selon la revendication 13, dans lequel la paroi des espaces de croissance de cellules est telle qu'aucune cellule dans aucun de ces espaces n'est éloignée de la membrane de plus d'environ 500 μm.

15. Module selon la revendication 3, dans lequel les canaux du premier moyen de circulation de fluide sont essentiellement parallèles aux canaux de croissance de cellules du deuxième moyen de circulation de fluide.

16. Module selon la revendication 3, dans lequel les canaux de transport de produit nutritif du premier moyen de circulation de fluide sont essentiellement perpendiculaires aux canaux de croissance de cellules du deuxième moyen de circulation de fluide.

17. Module selon la revendication 3, dans lequel la profondeur d'un espace de croissance de cellules est inférieure à environ 200 μm.

18. Module selon la revendication 3, dans lequel le seuio de poids moléculaire pour le passage à travers la membrane est situé entre environ 1000 et $10^7$ dalton.

19. Système selon la revendication 7, dans lequel le système est confiné et étanche.

20. Assemblage pour culture de cellules comprenant une structure rigide comportant une pluralité d'espaces de culture de cellules et une pluralité d'espaces séparés de milieu de culture, chaque espace de culture de cellules étant séparé du plus proche espace de milieu de culture par une membrane à perméabilité sélective, les espaces de culture de cellules simulant un réseau vasculaire et étant dimensionnés et orientés de telle manière que la distance entre une cellule située dans une région distale de l'un quelconque de ces espaces et le réseau vasculaire est inférieure ou essentiellement semblable à celle qui est typique des tissus ou organes dans lesquels on trouve in vivo une cellule du type cultivé.

FIG. 1

FIG. 2

FIG. 3

FIG. 4

FIG. 5

FIG. 6

FIG. 7

0 153 405

FIG. 8

0 153 405

FIG. 9

FIG. 10

FIG. II

FIG. 12  SHEET 40

FIG. 13  SHEET 41

FIG. 14  SHEET 42

FIG. 15  SHEET 43

FIG. 16  SHEET 44

FIG. 17  SHEET 45

0 153 405

SHEET 40

SHEET 41

SHEET 42

SHEET 43

SHEET 44

SHEET 41

SHEET 45

FIG. 18